(19) Europäisches Patentamt / European Patent Office / Office européen des brevets

(11) **EP 2 147 630 B1**

(12) **EUROPEAN PATENT SPECIFICATION**

(45) Date of publication and mention
of the grant of the patent:
**16.12.2015  Bulletin 2015/51**

(51) Int Cl.:
*A61B 1/00* *(2006.01)*   *A61B 1/01* *(2006.01)*
*A61B 1/018* *(2006.01)*   *A61B 17/00* *(2006.01)*
*A61B 17/28* *(2006.01)*   *A61B 18/14* *(2006.01)*
*A61B 19/00* *(2006.01)*

(21) Application number: **09008453.4**

(22) Date of filing: **29.06.2009**

(54) **Endoscopic surgical system**

Endoskopisches chirurgisches System

Système chirurgical endoscopique

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority:  **24.07.2008   JP 2008191041**

(43) Date of publication of application:
**27.01.2010   Bulletin 2010/04**

(73) Proprietor: **Olympus Corporation**
**Shibuya-ku**
**Tokyo (JP)**

(72) Inventor: **Yoshie, Michifumi**
**Hachioji-shi**
**Tokyo 192-8512 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
**EP-A- 2 064 984**      **WO-A-2006/016390**
**WO-A-2007/111571**    **US-A- 5 876 325**
**US-A1- 2008 027 279**

**Description**

The present disclosure relates to an endoscopic surgical system configured so as to perform surgery within the body of a patient while confirming the state of surgery performed by means of a surgical instrument through an endoscope image by inserting the surgical instrument into a channel of an endoscope and displaying a distal end portion of the surgical instrument within an observation field of the endoscope.

In general, a soft type endoscope includes an insertion portion to be inserted into a lumen. The insertion portion is provided with a flexible tube portion. A bending portion is disposed on a distal end side of the insertion portion and an operation portion on a near side of an operator is coupled to a proximal end portion of the insertion portion. The operation portion is provided with an operation knob for operating the bending portion in a bending manner. A surgical instrument insertion channel is formed inside of the insertion portion. A surgical instrument insertion portion is formed on a proximal end portion side of the insertion portion. The surgical instrument insertion portion is caused to communicate with a proximal end portion of the surgical instrument insertion channel.

A surgical instrument for an endoscope is inserted from the surgical instrument insertion portion into the surgical instrument insertion channel during surgery performed by means of the surgical instrument for an endoscope. The surgical instrument is configured so as to be inserted into, for example, the body of a patient through the surgical instrument insertion channel.

As an endoscopic surgical system, the following systems have been conventionally developed. That is, a distal end portion of a surgical instrument for an endoscope is displayed within an observation visual field of an endoscope in a state that the surgical instrument has been inserted from a surgical instrument insertion portion into a surgical instrument insertion channel. Thereby, surgery within the body of a patient is performed while the state of surgery performed by means of the surgical instrument is being confirmed with endoscope images.

Jpn. Pat. Appln. KOKAI Publication No. 2005-137701 describes an endoscope provided with a motorized bending portion and a motorized surgical instrument inserted into a surgical instrument insertion channel for an endoscope. The publication shows an endoscopic surgical system which performs procedure in combination of the endoscope provided with the motorized bending portion and the motorized surgical instrument inserted into the surgical instrument insertion channel for an endoscope.

Now, in a conventional endoscopic surgical system configured so as to perform surgery (procedure) within the body of a patient in combination of an endoscope and a surgical instrument to be inserted into the endoscope, the following problem arises. In the system, for example, when the endoscope is moved, the surgical instrument itself which has been inserted in the channel of the endoscope does not move. Therefore, there is a possibility that the position of the surgical instrument moves largely within an endoscope image. In this case, such a drawback may occur that the surgical instrument positioned within a visual field of the endoscope moves out of the visual field.

In the system disclosed in Jpn. Pat. Appln. KOKAI Publication No. 2005-137701 , for example, when the endoscope is moved, the position/attitude of the surgical instrument also changes according to position/attitude change of the endoscope. Therefore, the position/attitude change of the endoscope results in large change of position/attitude of the surgical instrument within the endoscope image.

US 5,876,325 discloses a surgical manipulator and a surgical device, wherein the position of the surgical manipulator is detected, the surgical device is moved accordingly, and a difference in the position of the surgical manipulator and the surgical device is detected such the that the position of the surgical device matches that of the surgical manipulator.

EP 2 064 984 discloses a manipulator drive section that is controlled so as to perform movement of a manipulator of a therapeutic device conforming to a manipulation of an operator even when a curved state of an insertion portion and a bending portion of an endoscope is changed. A curved state of a curving section of an insertion portion of an endoscope is detected, and, because the curved state of the insertion portion may affect the length of the wires which operate a curving state of the therapeutic device and a load on the wires for a curving operation, the amount of curving is accounted for when determining how much to operate the wires operable to curve the therapeutic tool in order that an amount of curving of the therapeutic device corresponds to an input of an operator independently of the curved state of the insertion portion.

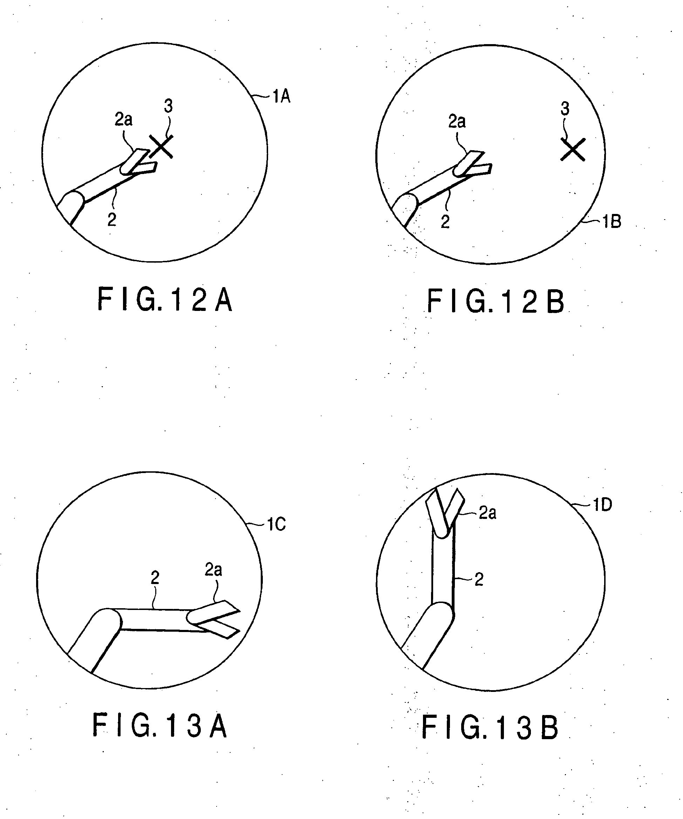

For example, an endoscope image 1A shown in FIG. 12A shows an observation state where a distal end portion 2a of a surgical instrument 2 is disposed at a position near an affected area 3. In this state, when a bending portion of an endoscope is bent in a left-hand direction, the endoscope image 1A shown in FIG. 12A changes like an endoscope image 1B shown in FIG. 12B. In the endoscope image 1B shown in FIG. 12B, a distance between the distal end portion 2a of the surgical instrument 2 and the affected area 3 becomes far. In an extreme case, the surgical instrument 2 positioned within the visual field of the endoscope image 1B may move out of the visual field.

An endoscope image 1C shown in FIG. 13A shows an observation state where the distal end portion 2a of the surgical instrument 2 inserted into the endoscope visual field from a lower direction of the endoscope visual field has been bent within the visual field in a right-hand direction by an angle of about 90[deg.]. In this state, when the insertion portion of the endoscope is rotated about an axis of the insertion portion in a counter clockwise direction by an angle of about 90[deg.], the endoscope image 1C shown in FIG. 13A changes like an endoscope image 1D shown in FIG. 13B. In the endoscope image 1C shown in FIG. 13B, the distal end portion 2a of the surgical instrument 2 is observed within the endoscope visual field such that it is bent in an upward direction. Therefore, such a drawback occurs that a relative position/attitude between the distal end portion 2a of the surgical instrument 2 and the affected area 3 changes.

In general, there is such a case that an operator desires changing of the endoscope visual field without causing the relative position/attitude of the distal end portion 2a of the surgical instrument 2 to the affected area 3 to change or causing the position/attitude of the distal end portion 2a of the surgical instrument 2 to change within the endoscope visual field. In the conventional endoscopic surgical system, however, the position/attitude of the endoscope cannot be operated without changing the relative position/attitude between the affected area and the surgical instrument in this manner. Therefore, for example, when the endoscope has been moved, such a drawback arises that the surgical instrument itself inserted in the endoscope does not moves but the position of the surgical instrument moves largely within the endoscope image, or the surgical instrument positioned within the endoscope visual field moves out of the endoscope visual field.

In view of these circumstances, it is an object to provide an endoscopic surgical system where, when procedure is performed, an endoscope and the position/attitude of a surgical instrument within an endoscope image can be operated independently from each other and the position/attitude of the endoscope can be operated without changing a relative position/attitude between an affected area and the surgical instrument.

An endoscopic surgical system according to claim 1 is provided. The endoscopic surgical system may comprise an endoscope; a surgical instrument which can be inserted into a channel of the endoscope or a channel of an overtube attached to the endoscope and which is used for surgery on an analyte; surgical instrument position/attitude information acquisition means for acquiring position/attitude information of the surgical instrument, surgical instrument powering means for actively actuating the surgical instrument, control means for controlling the surgical instrument powering means, and surgical instrument instruction input means for transmitting an instruction input signal to the control means, wherein endoscope position/attitude information acquisition means which is connected to the control means and acquires position/attitude information of the endoscope is provided, and the control means includes control signal adjusting means for adjusting a control signal of the surgical instrument powering means utilizing the position/attitude information.

With the above configuration, the surgical instrument powering means for actively actuating the surgical instrument is assembled in the system, the surgical instrument where the surgical instrument powering means is controlled by the control means is inserted in the channel of the endoscope or in the channel of the overtube attached to the endoscope, and the surgical instrument is inserted into the body of a patient through the channel to be used during surgery on an analyte. At this time, an instruction input signal is fed to the control means by the surgical instrument instruction input means and the position/attitude information of the surgical instrument is acquired by the surgical instrument position/attitude information acquisition means. Further, the position attitude information of the endoscope is acquired by the endoscope position/attitude information acquisition means connected to control means. The control means utilizes the position/attitude information to cause the control signal of the surgical instrument powering means. Thereby, influence to the position/attitude of the surgical instrument within the endoscope image from operation of the endoscope can be cancelled, so that, when an operator performs a procedure, he/she can operate the endoscope and the position/attitude of the surgical instrument within the endoscope image independently from each other and he/she can operate the endoscope position/attitude without changing the relative position/attitude between the affected area and the surgical instrument.

According to another aspect, there is provided an endoscopic surgical system comprising an endoscopic system and a surgical system, where the endoscopic system comprises an endoscope, an endoscope position/attitude information acquisition device for acquiring position/attitude information of an insertion portion of the endoscope, endoscope powering means for straightly moving and rotating the insertion portion of the endoscope, endoscope control means for controlling the endoscope powering means, and endoscope instruction input means for transmitting an instruction input signal to the endoscope control means; and the surgical system comprises a surgical instrument which can be inserted in a channel of the endoscope or a channel of an overtube attached to the endoscope and which is used for surgery on an analyte, surgical instrument position/attitude information acquisition means for acquiring position/attitude information of the surgical instrument, surgical instrument powering means for actively actuating the surgical instrument, surgical instrument control means for controlling the surgical instrument powering means, surgical' instrument instruction input means for transmitting an instruction input signal to the surgical instrument control means,

and surgical instrument control means for controlling the endoscope powering means and the surgical instrument powering means, wherein the surgical system performs communication with the endoscopic system regarding the endoscope position/attitude information, and the surgical instrument control means controls the surgical instrument powering means while causing the surgical instrument powering means to cooperate with the endoscope powering means.

In the endoscopic surgical system with the abovementioned configuration, the surgical instrument powering means for actively actuating the surgical instrument is assembled, the surgical instrument where the surgical instrument powering means is controlled by the control means is inserted in the channel of the endoscope or in the channel of the overtube attached to the endoscope, and the surgical instrument is inserted into the body of a patient through the channel to be used during surgery on an analyte. At this time, the endoscopic system and the surgical system are driven, respectively. An instruction input signal is transmitted to the endoscope control means by the endoscope instruction input means at a driving time of the endoscopic system. The endoscope powering means is controlled by the endoscope control means, and an insertion portion of the endoscope is moved straightly and rotated by the endoscope powering means. At this time, position/attitude information of the insertion portion of the endoscope is acquired by the endoscope position/attitude information acquisition apparatus. Further, at a driving time of the surgical system, an instruction input signal is transmitted to the surgical instrument control means by the surgical instrument instruction input means, the surgical instrument powering means is controlled by the surgical instrument control means, and the surgical instrument is actively actuated by the surgical instrument powering means. At this time, position/attitude information of the insertion portion of the endoscope is acquired by the endoscope position/attitude information acquisition apparatus. Further, at a driving time of the surgical system, an instruction input signal is transmitted to the surgical instrument control means by the surgical instrument instruction input means, the surgical instrument powering means is controlled by the surgical instrument control means, and the surgical instrument is actively actuated by the surgical instrument powering means. At this time, position/attitude information of the surgical instrument is acquired by the surgical instrument position/attitude information acquisition means connected to the surgical instrument control means. Further, the surgical system communicates with the endoscopic system regarding endoscope position/attitude information, and the surgical instrument control means controls the surgical instrument powering means while causing the surgical instrument powering means to cooperate with the endoscope powering means. Thereby, influence to the position/attitude of the surgical instrument within the endoscope image from operation of the endoscope can be cancelled, so that when an operator performs procedure, he/she can operate the endoscope and the position/attitude of the surgical instrument within the endoscope image independently from each other and he/she can operate the endoscope position/attitude without changing a relative position/attitude between the affected area and the surgical instrument.

According to still another aspect, there is provided an endoscopic surgical system comprising an endoscopic system and a surgical system, where the endoscopic system comprises an endoscope having an active bending portion at a distal end portion of an insertion portion to be inserted into a lumen, an endoscope position/attitude information acquisition device for acquiring position/attitude information of the insertion portion of the endoscope, endoscope powering means which straightly moves and rotates the insertion portion of the endoscope, an endoscope bending information acquisition device for acquiring bending information of the bending portion, bending portion powering means for actuating the active bending portion of the endoscope in a bending manner, endoscope control means for controlling the endoscope powering means and the bending portion powering means, and endoscope instruction input means for transmitting an instruction input signal to the endoscope control means; and the surgical system comprises a surgical instrument which can be inserted into a channel of the endoscope or a channel of an overtube attached to the endoscope and which is used for surgery on an analyte, surgical instrument position/attitude information acquisition means for acquiring position/attitude information of the surgical instrument, surgical instrument powering means for actively actuating the surgical instrument, surgical instrument control means for controlling the surgical instrument powering means, surgical instrument instruction input means for transmitting an instruction input signal to the surgical instrument control means, and surgical instrument control means for controlling the endoscope powering means and the surgical instrument powering means, wherein the surgical system performs communication with the endoscopic system regarding the endoscope position/attitude information, and the surgical instrument control means controls the surgical instrument powering means while causing the surgical instrument powering means to cooperate with the endoscope powering means.

In the endoscopic surgical system with the abovementioned configuration, the surgical instrument powering means for actively actuating the surgical instrument is assembled, and the surgical instrument where the surgical instrument powering means is controlled by the control means and the endoscope having the active bending portion at the distal end portion of the insertion portion, where the active bending portion is actuated in a bending manner by the bending portion powering means, are used. In this case, the surgical instrument is inserted in the channel of the endoscope or in the channel of the overtube attached to the endoscope:and it is inserted into the body of a patient through the channel to be used during surgery on an analyte. At this time, the endoscopic system and the surgical system are driven, respectively. At a driving time of the endoscopic system, an instruction input signal is transmitted to the endoscope control means by the endoscope instruction input means, and the endoscope powering means and the bending portion powering means are controlled by the endoscope control means. The insertion portion of the endoscope is straightly moved and rotated by the endoscope powering means, and the active bending portion of the endoscope is actuated in a bending manner by the bending portion powering means. At this time, position/attitude information of the insertion portion of the endoscope is acquired by the endoscope position/attitude information acquisition device, and bending information of the bending portion is acquired by the endoscope bending information acquisition device. At a driving time of the surgical system, an instruction input signal is transmitted to the surgical instrument control means by the surgical instrument instruction input means, so that the surgical instrument powering means is controlled by the surgical instrument control means. At this time, the surgical instrument is actively actuated by the surgical instrument powering means, and position/attitude information of the surgical instrument is acquired by the surgical instrument position/attitude information acquisition means. Further, the surgical system performs communication with the endoscopic system regarding the endoscope position/attitude information and the surgical instrument control means controls the surgical instrument powering means while causing the surgical instrument powering means to cooperate with the endoscope powering means. Thereby, influence to the position/attitude of the surgical instrument within an endoscope image from operation of the endoscope can be cancelled, so that, when an operator performs procedure, he/she can operate the endoscope and the position/attitude of the surgical instrument within the endoscope image independently from each other and he/she can operate the position/attitude of the endoscope without changing a relative position/attitude between an affected area and the surgical instrument.

Since actuation can be performed while movement of the endoscope and movement of the surgical instrument are caused to cooperate with each other, the endoscope and the position/attitude of the surgical instrument within an endoscope image can be operated independently from each other when procedure is performed. According to the control method, the position/attitude of the endoscope can be operated without changing a relative position/attitude between an affected area and the surgical instrument.

The invention can be more fully understood from the following detailed description when taken in conjunction with the accompanying drawings, in which:

FIG. 1A is a schematic configuration diagram of a whole endoscopic surgical system according to a first embodiment;

FIG. 1B is a front diagram showing a distal end face of an endoscope used in the endoscopic surgical system;

FIG. 2 is a schematic configuration diagram of a whole surgical instrument of the endoscopic surgical system according to the first embodiment;

FIG. 3 is a schematic configuration diagram of a main section showing endoscope position/attitude information acquisition means in the endoscopic surgical system according to the first embodiment;

FIG. 4 is a block diagram showing a schematic configuration of the whole endoscopic surgical system

according to the first embodiment;

FIG. 5 is a schematic configuration diagram of a main section showing a first modified example of the endoscope position/attitude information acquisition means in the endoscopic surgical system according to the first embodiment;

FIG. 6 is a schematic configuration diagram of a main section showing a second modified example of the endoscope position/attitude information acquisition means in the endoscopic surgical system according to the first embodiment;

FIG. 7 is a schematic configuration diagram of a whole endoscopic surgical system according to a second embodiment;

FIG. 8 is a schematic configuration diagram of a main section showing one example of a powering apparatus of an endoscope of the endoscopic surgical system according to the second embodiment;

FIG. 9 is a block diagram showing a schematic configuration of the whole endoscopic surgical system according to the second embodiment;

FIG. 10 is a schematic configuration diagram of a whole endoscopic surgical system according to a third embodiment;

FIG. 11 is a block diagram showing a schematic configuration of the whole endoscopic surgical system according to the third embodiment;

FIG. 12A is a plan view showing a state where a distal end portion of a motorized surgical instrument and an affected area have been displayed within an observation image of an endoscope in an insertion state of the motorized surgical instrument into a channel of a conventional endoscope;

FIG. 12B is a plan view showing a state where the orientation of a bending portion of the conventional endoscope has been changed in a left-hand direction in the state shown in FIG. 12A;

FIG. 13A is a plan view showing a state where the distal end portion of the motorized surgical instrument displayed within the visual field of the endoscope has been bent in a direction of three o'clock by an angle of 90[deg.] in the insertion state of the motorized surgical instrument into the channel of the conventional endoscope; and

FIG. 13B is a plan view showing an endoscope image obtained when the endoscope has been rotated in a counter clockwise direction by an angle of 90[deg.] from the state shown in FIG. 13A.

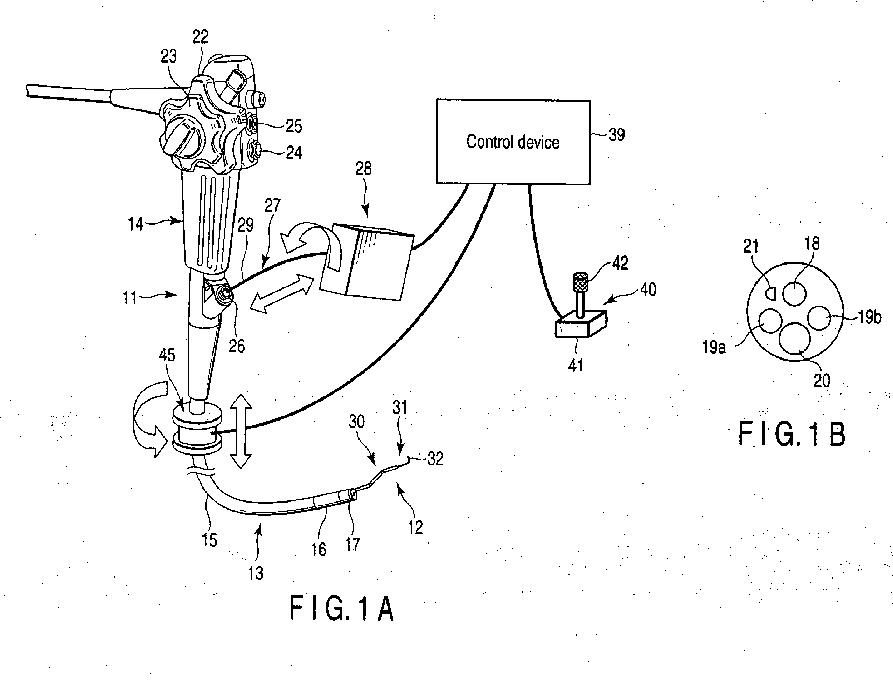

A first embodiment will be explained below with reference to FIGS. 1A to 4. FIG. 1A is a schematic configuration diagram of a whole endoscopic surgical system according to the first embodiment. In the endoscopic surgical system according to the embodiment, an endoscope 11 and a surgical instrument 12 for surgery on an analyte are used in combination.

The endoscope 11 includes an elongated insertion portion 13 inserted into the body of a patient and an operation section 14 coupled to a proximal end portion of the insertion portion 13. The insertion portion 13 includes an elongated flexible tube portion 15, a bending portion 16 coupled to a distal end of the flexible tube portion 15, and a distal end hard portion 17 coupled to a distal end of the bending portion 16. The bending portion 16 can be operated in a bending manner, for example, in four directions of upward and downward directions and leftward and rightward directions.

As shown in FIG. 1B, a distal end face of the distal end hard portion 17 is provided with, for example, one observation window portion 18, two illumination window portions 19a and 19b, an opening of a channel 20 for insertion of one surgical instrument, and one gas-feeding and water-feeding nozzle 21. An imaging section provided with an optical system such as an objective lens (not shown) and an imaging element such as CCD is disposed inside the observation window portion 18. A lesion or the like within the body cavity is imaged by the imaging section. An imaging signal obtained at the imaging section of the endoscope 11 is transmitted to a display processor (not shown) through a connection cable to be converted to a video signal and an image imaged at the endoscope 11 is displayed on a display device (not shown) by the video signal.

The operation section 14 are provided with an upward and downward direction bending operation knob 22 for operating the bending portion 16 in bending manner in upward and downward directions, a leftward and rightward direction bending operation knob 23 for operating the bending portion 16 in bending manner in leftward and rightward directions, a gas-feeding and water-feeding button 24, a suction button 25, various switches for imaging operation, and the like. Further, a channel port 26 communicating with the channel 20 for surgical instrument insertion is formed near a coupling portion of the operation section 14 and the insertion portion 13. The surgical instrument 12 is inserted into the channel port 26.

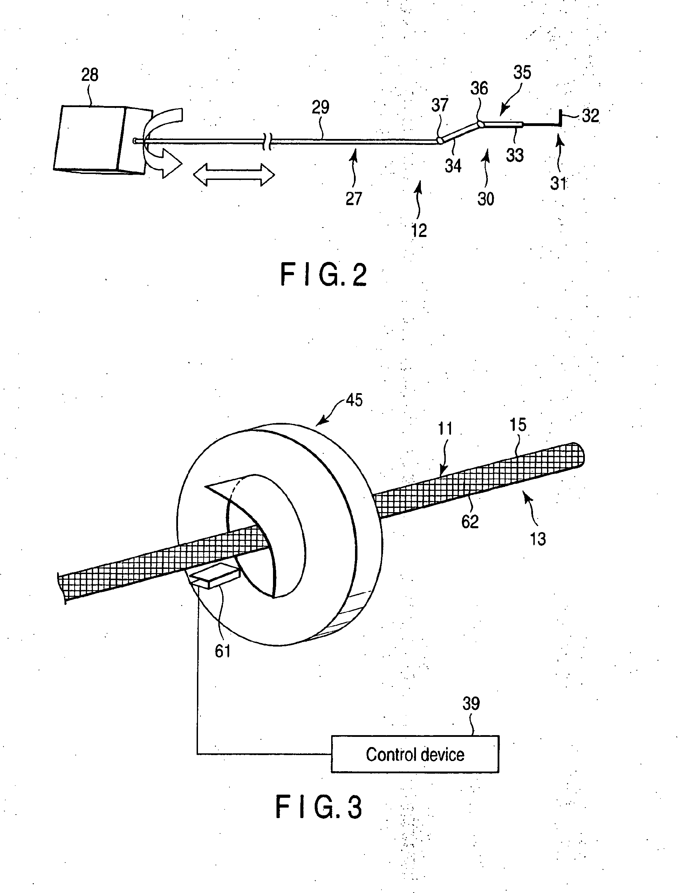

The surgical instrument 12 according to the embodiment comprises an active surgical instrument (motorized surgical instrument) driven by a motor. As shown in FIG. 2, the active surgical instrument 12 has an elongated insertion portion 27 inserted into the channel 20 for surgical instrument insertion of the endoscope 11. A proximal end portion of the insertion portion 27 is coupled with a motor box (surgical instrument powering means) 28. The insertion portion 27 comprises an elongated flexible tube (soft portion) 29 positioned at a near side of an operator, a bending portion 30 connected to a distal end of the flexible tube 29, and a distal end surgical portion 31 connected to a distal end of the bending portion 30.

The flexible tube 29 is a flexible portion which can be bent relatively softly and elastically by external force. The bending portion 30 is a portion which is forcibly bent by the motor box 28. The distal end surgical portion 31 is provided with a surgical function corresponding to the active surgical instrument 12. In the embodiment, a high-frequency knife 32 is provided, for example, as one example of the surgical function.

As shown in FIG. 2, the bending portion 30 is provided with an articulated bending mechanism 35 comprising a plurality of bending pieces (joint pieces), two bending pieces 33 and 34 in this embodiment. The bending pieces 33 and 34 are each formed using an annular member. The respective bending pieces 33 and 34 are arranged coaxially in a line in an axial direction of the insertion portion 27. These pieces are called a first bending piece 33 and a second bending piece 34 from a distal end in this order.

The distal end surgical portion 31 is fixed on a front end portion of the first bending pierce 33. A rear end portion of the first bending piece 33 is foldably coupled to a front end portion of the second bending piece 34 via a first joint portion (folding portion) 36. A rear end portion of the second bending piece 34 is foldably coupled to a front end portion of the flexible tube 29 via a second joint portion (folding portion) 37.

In the embodiment, wire units (not shown) for folding the first bending piece 33 and the second bending piece 34 individually are provided within the insertion portion 27. Each wire unit comprises a pair of non-stretchable operation wires as one set.

The first bending piece 33 is driven by two operation wires of a first set of wire unit. Similarly, the second bending piece 34 is driven by two operation wires of a second set of wire unit.

The respective operation wires are inserted in individual flexible guide sheaths movably in advancing and retreating directions. Each guide sheath is made of a sheath-like flexible member such as, for example, a close coil or a resin tube. Only advancing and retreating directions of each operation wire are guided by an inner hole of the guide sheath of the flexible member.

The respective guide sheaths are led up to the motor box 28 positioned on the near side of the operator. Thereby, the respective operation wires are individually led up to the motor box 28 on the near side of the operator through insides of the individual flexible guide sheaths. The first bending piece 33 and the second bending piece 34 can be individually rotated by individually and independently driving two sets of wire units. That is, only the first bending piece 33 is individually and independently rotated about the first joint portion 36 to be individually folded by pushing or pulling two operation wires of the first set of wire units. Similarly, only the second bending piece 34 can be individually and independently folded about the second joint portion 37 by two operation wires of the second set of wire units. Thereby, the articulated bending mechanism 35 where two joints of the first joint portion 36 and the second joint portion 37 can be driven individually and independently is configured in this embodiment. The articulated bending mechanism 35 is covered with a soft outer skin (not shown) so that all members thereof configure the bending portion 30.

Incidentally, in the embodiment, an example where the articulated bending mechanism 35 comprises two bending pieces 33 and 34 has been shown, but the number of bending pieces is not limited to two. For example, the articulated bending mechanism 35 may comprise at least three bending pieces. In the embodiment, the articulated bending mechanism 35 is configured using two bending pieces 33 and 34, but it may be configured using a bending link mechanism instead of the bending pieces 33 and 34.

A bending portion operation mechanism (not shown) rotationally driving the first bending piece 33 and the second bending piece 34 of the bending portion 30 individually, a motor (not shown) actuating the surgical instrument 12 in an inserting direction of the surgical instrument 12, and a motor (not shown) rotationally driving the surgical instrument 12 in a spinning direction are accommodated in the motor box 28 of the active surgical instrument 12. The bending portion operation mechanism is provided with two driving motors (not shown) performing pushing and pulling operations of two sets of operation wires corresponding to the first bending piece 33 and the second bending piece 34 to be rotationally operated. Two sets of operation wires are pushed and pulled by driving two driving motors individually.

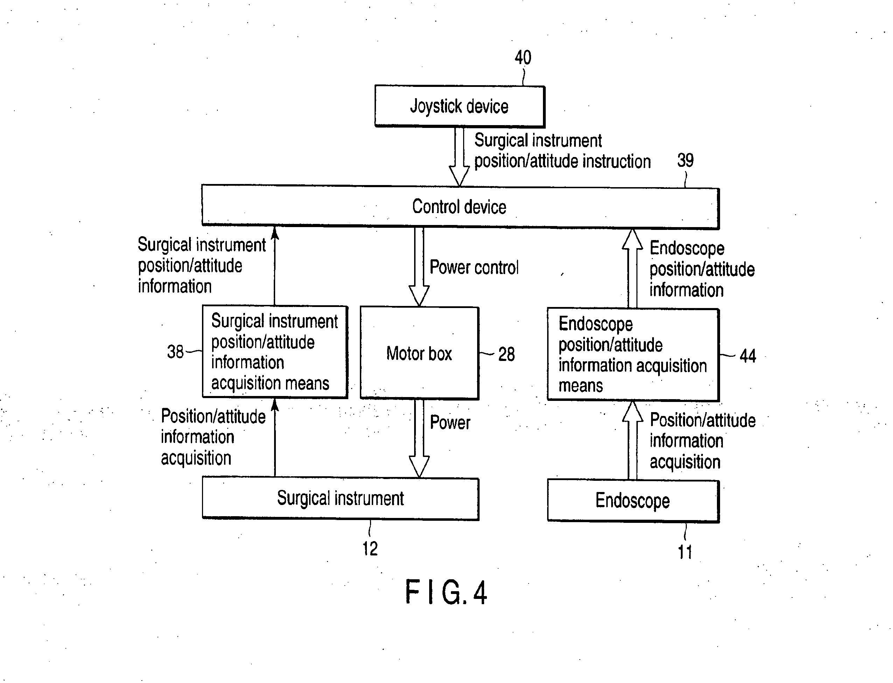

As shown in FIG. 4, the endoscopic surgical system according to the embodiment is provided with surgical instrument position/attitude information acquisition means 38 for acquiring position/attitude information of the surgical instrument 12, the motor box 28 serving as surgical instrument powering means actively actuating the surgical instrument 12, a rotation and pushing-in control device (control means) 39 controlling the motor box 28, a joystick device (surgical instrument instruction input means) 40 transmitting an instruction input signal to the rotation and pushing-in control device 39, and an endoscope position/attitude information acquisition means 44 acquiring position/attitude information of the endoscope 11. The surgical instrument position/attitude information acquisition means 38 may be an encoder or a potentiometer directly sensing joint positions of the first joint portion 36 and the second joint portion 37 of the bending portion 30. Incidentally, sensing can be performed by measuring a movement amount of the wire connected to the joint position.

As shown in FIG. 1A, the joystick device 40 includes a base member 41 and a joystick 42 provided on an upper face of the base member 41 so as to allow a tilting operation, a pushing-in operation, and a rotating operation about an axis thereof. An operation signal for operating the joystick 42 on the upper face of the base member 41 is input into the control device 39. Here, a surgical instrument operation of the joystick device 40 is performed in the following manner. That is, a bending operation of the bending portion 30 of the surgical instrument 12 is performed by a tilting operation of the joystick 42, insertion of the surgical instrument 12 is performed by a pushing-in operation of the joystick 42, and a rotating operation of the surgical instrument 12 about an axis is performed by a rotating operation of the joystick 42 about an axis. Actuation of the motor box 28 is controlled in response to the input signal by the control device 39, so that the bending portion 30 of the surgical instrument 12 is remotely operated in a bending manner.

As shown in FIG. 3, the endoscope position/attitude information acquisition means 44 is configured by assembling a photoreflector 61 in a mouthpiece 45. The photoreflector 61 is connected to the control device 39.

Further, as the endoscope 11, an endoscope with the insertion portion 13 having a stripe pattern 62 on an outer peripheral face thereof is utilized. Thereby, movement of the stripe pattern 62 according to movement of the insertion portion 13 of the endoscope 11 is sensed by the photoreflector 61 so that an insertion amount and a rotation amount about an axis of the insertion portion 13 of the endoscope 11 are detected.

Next, an operation of the endoscopic surgical system with the abovementioned configuration according to the embodiment will be explained. The following behaviors or steps are performed at the time of use of the endoscopic surgical system according to the embodiment.

Step 1: An affected area is observed by the endoscope 11.

Step 2: The insertion portion 27 of the surgical instrument 12 is inserted into the channel 20 of the endoscope 11 so that surgical instrument control is started.

Step 3-1: An operator operates the joystick 42 of the joystick device 40 in a tilting manner, thereby inputting an instruction for operating the bending portion 30 of the surgical instrument 12 in a bending manner.

Step 3-2: The joystick device 40 transmits an instruction input signal to the control device 39. Thereby, the first bending piece 33 and the second bending piece 34 of the bending portion 30 are rotationally driven individually by the bending portion operation mechanism of the motor box 28 so that the bending portion 30 of the surgical instrument 12 is operated in a bending manner in response to an tilting operation of the joystick 42.

Step 4: Position/attitude information of the surgical instrument 12 is acquired by the surgical instrument position/attitude acquisition means 38 at a bending operation time of the bending portion 30 of the surgical instrument 12. The surgical instrument position/attitude information is transmitted from the surgical instrument position/attitude acquisition means 38 to the control device 39.

Step 5-1: The operator operates the insertion portion 13 of the endoscope 11 in a rotating or an advancing or retreating direction in order to change the visual field of the endoscope 11.

Step 5-2: Position/attitude information of the insertion portion 13 of the endoscope 11 is acquired by an insertion amount detecting section 50 and a rotation amount detecting section 51 of the endoscope position/attitude information acquisition means 44 to be transmitted to the control device 39 at an operation time of the insertion portion 13 of the endoscope 11.

Step 6: At this time, the control device 39 performs calculation based upon the respective signals received at the above Steps 3-2, 4, and 5-2 to control power of the motor box 28. Thereby, control for adjusting a control signal of the motor box 28 is performed utilizing position/attitude information from the endoscope position/attitude information acquisition means 44.

Step 7: Thereafter, power generated from the motor box 28 is transmitted to the bending portion 30 of the surgical instrument 12 so that the bending portion 30 of the surgical instrument 12 is actuated.

The abovementioned Steps 3-1 to 7 configure a series of behaviors or steps. Here, control in Step 6 is control for cancelling influence to position/attitude of the bending portion 30 of the surgical instrument 12 within an endoscope image from operation of the insertion portion 13 of the endoscope 11 in Step 5-1. Thereby, an operator can operate the endoscope 11 and the surgical instrument 12 as devices independent from each other.

Accordingly, the following effect can be obtained according to the abovementioned configuration. That is, in the system of the endoscope apparatus 1 according to the embodiment, the position information of the insertion portion 13 of the soft endoscope 11 is incorporated for control of the active surgical instrument 12 inserted into the channel 20 of the endoscope 11 thereof. For example, movement of the stripe pattern 62 according to movement of the insertion portion 13 of the endoscope 11 is sensed by the photoreflector 61 of the endoscope position/attitude information acquisition means 44. Thereby, an insertion amount and a rotation amount about an axis of the insertion portion 13 of the endoscope 11 are sensed. At this time, when an insertion amount x and a rotation amount $\alpha$ of the insertion portion 13 of the endoscope 11 are detected based upon a detection signal from the photoreflector 61, behavior or actuation of the motor box 28 is controlled in the following manner by the control device 39 at a control time of the active surgical instrument 12. That is, instructions about an insertion amount y and a rotation amount $\beta$ of the surgical instrument 12 are received according to a pushing-in operation and a rotation operation of the joystick 42 of the joystick device 40. When the insertion amount of the insertion portion 13 of the soft endoscope 11 changes to x and the rotation amount thereof changes to $\alpha$, behavior or actuation of the motor box 28 is controlled by the control device 39 such that the surgical instrument 12 is moved by an insertion amount z and it is rotated by a rotation amount $\gamma$. At this time, the control device 39 controls behavior or actuation of the motor box 28 such that the insertion amount z of the surgical instrument 12 and the rotation amount $\gamma$ thereof satisfy the following equations.

$$z = y - x$$
$$\gamma = \beta - \alpha$$

Thereby, position deviation of the active surgical instrument 12 occurring when the position of the soft endoscope 11 has changed can be eliminated. Accordingly, improvement of behavior or actuation efficiency and position accuracy of the active surgical instrument 12 can be achieved. As a result, an endoscopic surgical system where, when procedure is performed, the endoscope 11 and position/attitude of the surgical instrument 12 within an endoscope image can be operated independently from each other and position/attitude of the endoscope 11 can be operated without changing a relative position/attitude between an affected area and the surgical instrument 12 can be provided.

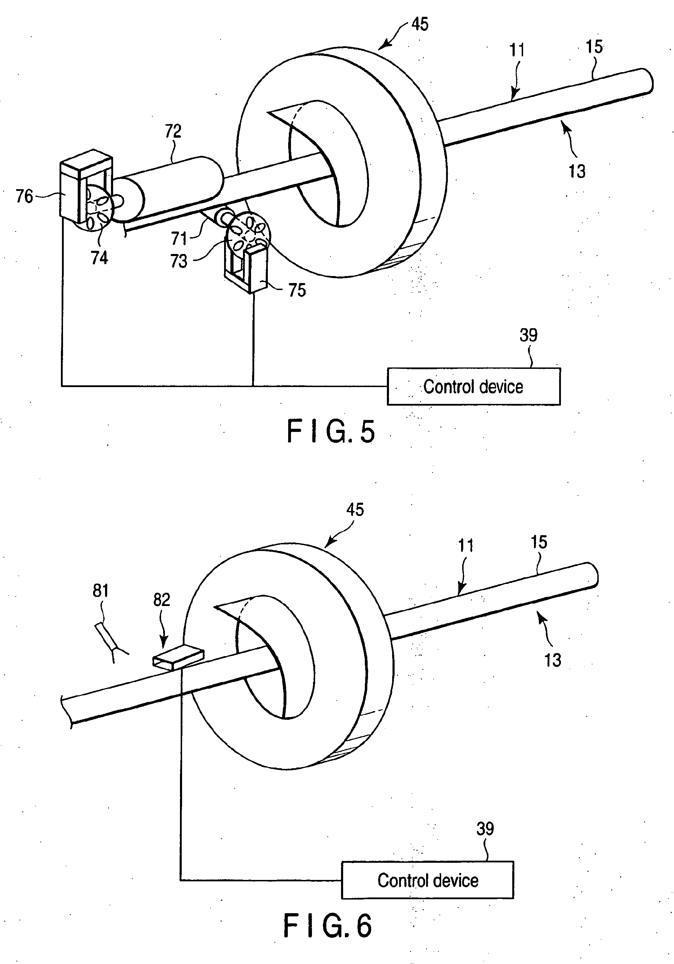

FIG. 5 is a schematic configuration diagram of a main section showing a first modified example of the endoscope position/attitude information acquisition means 44 of the endoscopic surgical system according to the first embodiment. In the first embodiment, the configuration where the photoreflector 61 is assembled to the mouthpiece 45 has been shown. In the modified example, the following configuration is adopted instead of the configuration in the first embodiment.

That is, in the modified example, two (first and second) rollers 71 and 72 coming in contact with the insertion portion 13 of the endoscope 11 when the insertion portion 13 of the endoscope 11 is inserted into the mouthpiece 45 is assembled in the mouthpiece 45. The first roller 71 is supported so as to rotate according to movement of the insertion portion 13 of the endoscope 11 in the axial direction. The second roller 72 is supported so as to rotate according to rotational motion of the insertion portion 13 of the endoscope 11 in a spinning of the insertion portion 13. Thereby, the respective rollers 71 and 72 are rotated according to movement of the insertion portion 13 of the endoscope 11 in the inserting direction and rotational motion thereof in the rotational direction.

The first roller 71 is provided with a first bored wheel 73 and the second roller 72 is provided with a second bored wheel 74. Further, rotation of the first bored wheel 73 is detected by a first photointerrupter 75, while rotation of the second bored wheel 74 is detected by a second photointerrupter 76, respectively. The first photointerrupter 75 and the second photointerrupter 76 are connected to the control device 39.

The first roller 71 rotates according to movement of the insertion portion 13 of the endoscope 11 in the axial direction so that rotation of the first bored wheel 73 rotating together with the first roller 71 is sensed by the first photointerrupter 75. Further, the second roller 72 rotates according to rotational motion of the insertion portion 13 of the endoscope 11 in a spinning direction of the insertion portion 13 so that rotation of the second bored wheel 74 rotating together with the second roller 72 is sensed by the second photointerrupter 76. Thereby, the insertion amount of the insertion portion 13 of the endoscope 11 and the rotation amount thereof in a spinning direction can be detected.

FIG. 6 is a schematic configuration diagram of a main section showing a second modified example of the endoscope position/attitude information acquisition means 44 of the endoscopic surgical system according to the first embodiment. In the modified example, light is shed on the insertion portion 13 of the endoscope 11 by an LED light source 81 and reflection thereof is measures by a light receiving sensor 82. Undulation or a pattern of the insertion portion 13 of the endoscope 11 is sensed by the reflection of light, so that the insertion amount and the rotation amount of the insertion portion 13 of the endoscope 11 are detected.

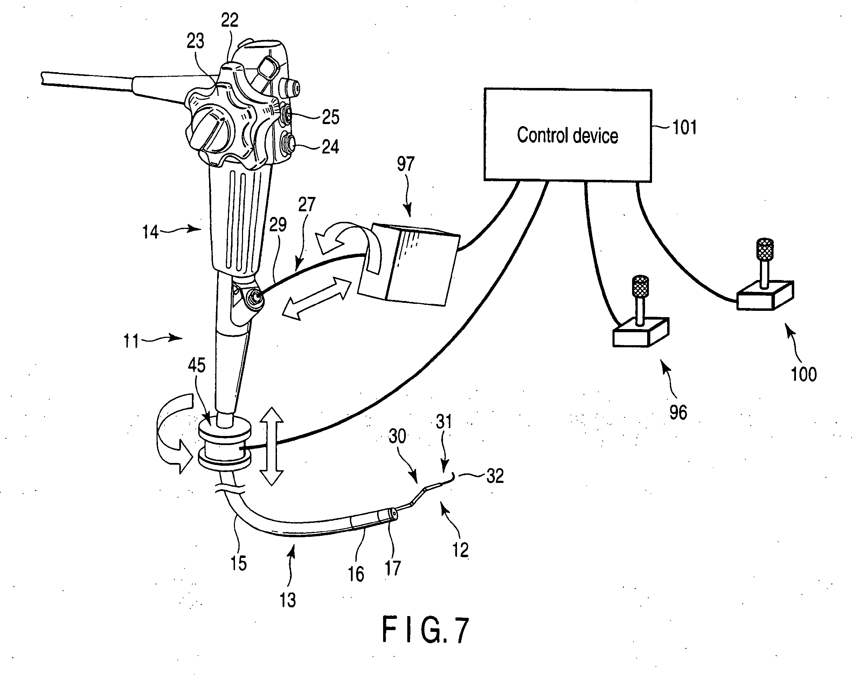

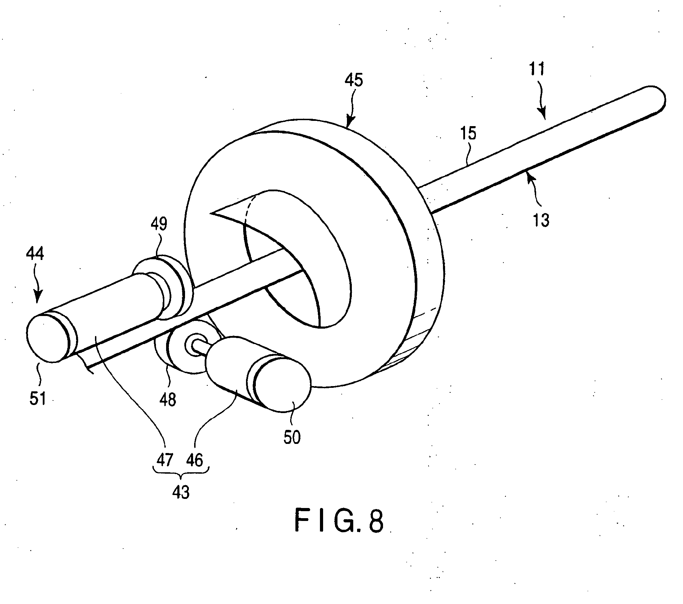

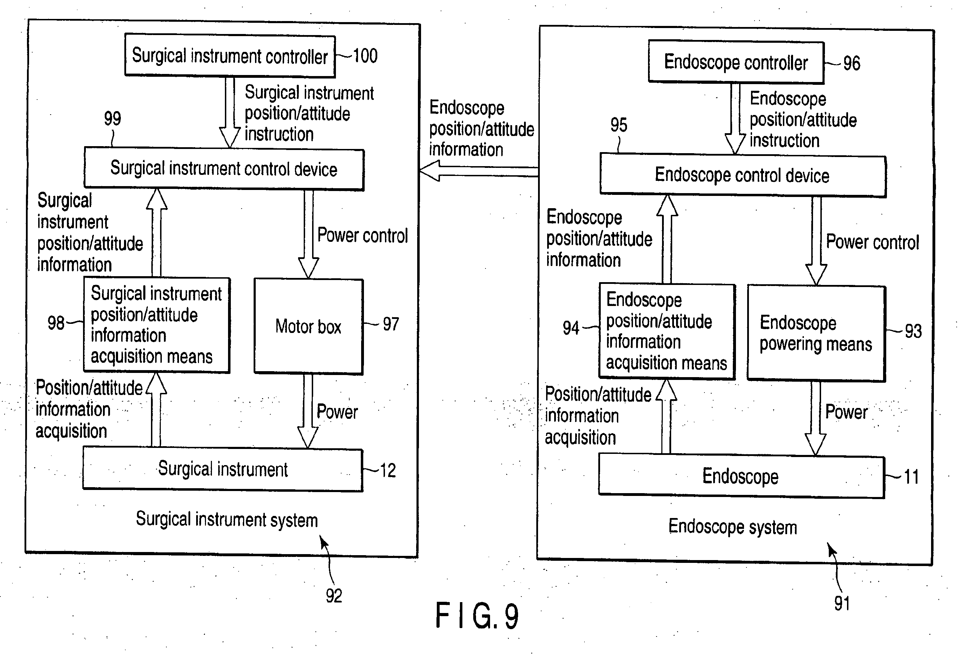

FIGS. 7 to 9 show a second embodimen. FIG. 7 is a schematic configuration diagram of a whole endoscopic surgical system according to the second embodiment, FIG. 8 is a schematic configuration diagram of one example of an endoscope driving device 43 described later, and FIG. 9 is a block diagram of the whole endoscopic surgical system. Incidentally, in the second embodiment, same parts or portions as those of the first embodiment are attached with same reference numerals, and explanation thereof is omitted.

As shown in FIG. 9, the endoscopic surgical system according to the second embodiment includes an endoscopic system 91 driving the endoscope 11 of the first embodiment (see FIGS. 1 to 4) and a surgical instrument system 92 driving the active surgical instrument 12 of the first embodiment.

The endoscopic system 91 includes the endoscope 11, endoscope powering means 93, endoscope position/attitude information acquisition means 94 acquiring position/attitude information of the endoscope 11, an endoscope control device (endoscope control means) 95, and an endoscope controller (endoscope instruction input means) 96.

The endoscope powering means 93 performs straight movement and rotational movement of the insertion portion 13 of the endoscope 11. That is, the endoscope powering means 93 performs inserting and detaching operations of the insertion portion 13 for moving the insertion portion 13 of the endoscope 11 in an axial direction of the insertion portion 13 and rotating operation of the insertion portion 13 of the endoscope 11 in a spinning direction of the insertion portion 13.

FIG. 8 shows one example of the endoscope driving device 43 which is endoscope powering means 93 driving the endoscope 11. Here, the endoscope driving device 43 includes two (first and second) driving motors 46 and 47, an axial direction driving roller 48, and a spinning direction driving roller 49. The first and second driving motors 46 and 47 are assembled to a mouthpiece 45 put in a mouth of a patient for attachment. The axial direction driving roller 48 is fixed to a rotational shaft of the first driving motor 46. The spinning direction driving roller 49 is fixed to a rotational shaft of the second driving motor 47. The first and second driving motors 46 and 47 are connected to the control device 39.

The axial direction driving roller 48 performs behavior or actuation for moving the insertion portion 13 of the endoscope 11 inserted into the mouthpiece 45 in an axial direction of the insertion portion 13 according to rotation of the first driving motor 46, namely, inserting and detaching operations of the insertion portion 13 of the endoscope 11. The spinning direction driving roller 49 performs behavior for rotating the insertion portion 13 of the endoscope 11 inserted into the mouthpiece 45 in a spinning direction of the insertion portion 13 according to rotation of the second driving motor 47.

The endoscope position/attitude information acquisition means 94 includes an insertion amount detecting section 50 and a rotation amount detecting section 51. The insertion amount detecting section 50 detects an insertion amount of the insertion portion 13 of the endoscope 11. The rotation amount detecting section 51 detects a rotation amount of the insertion portion 13 of the endoscope 11 in the spinning direction thereof. The insertion amount detecting section 50 includes an encoder (not shown) detecting a rotation amount of the first driving motor 46. The rotation amount detecting section 51 includes an encoder (not shown) detecting a rotation amount of the second driving motor 47. The insertion amount detecting section 50 and the rotation amount detecting section 51 are connected to the endoscope control device 95.

The endoscope position/attitude information acquisition means 94 detects an insertion amount x and a rotation amount α of the insertion portion 13 of the soft endoscope 11 based upon detection signals from the respective encoders of the insertion amount detecting section 50 and the rotation amount detecting section 51. Thereby, position/attitude information of the insertion portion 13 of the soft endoscope 11 is detected by the endoscope position/attitude information acquisition means 94. At this time, the endoscope control device 95 includes control signal adjusting means which adjusts a control signal of the motor box 97 utilizing the position/attitude information from the endoscope position/attitude information acquisition means 94. For example, when instructions about an insertion amount y and a rotation amount β of the surgical instrument 12 are received according to rotating and pushing-in operations of the joystick 42 of the joystick device 40, behavior or actuation of the motor box 97 is controlled by the surgical instrument control device 99 such that the surgical instrument 12 is inserted by an insertion amount z and rotated by a rotation amount γ. At this time, the surgical instrument control device 99 controls behavior of the motor box 97 such that the insertion amount z of the surgical instrument 12 and the rotation amount γ thereof satisfy the following equations.

$$z = y - x$$
$$\gamma = \beta - \alpha$$

The endoscope controller 96 may comprise a joystick device, for example. Further, the endoscope control device 95 is connected with the endoscope controller 96, the endoscope powering means 93, and the endoscope position/attitude information acquisition means 94, respectively. An instruction input signal is transmitted to the endoscope control device 95 according to operation of the endoscope controller 96 by an operator. The endoscope powering means 93 is controlled by endoscope control device 95 based upon the instruction input signal, so that behavior for straightly moving and rotating the insertion portion 13 of the endoscope 11 is performed. At this time, position/attitude information of the insertion portion 13 of the endoscope 11 is acquired by the endoscope position/attitude information acquisition means 94.

The surgical instrument system 92 is provided with the surgical instrument 12, a motor box 97 having the same configuration as that of the motor box 28 of the first embodiment, surgical instrument position/attitude information acquisition means 98 for acquiring position/attitude information of the surgical instrument 12, a surgical instrument control device (surgical instrument control means) 99 controlling the endoscope powering means 93 and the motor box 97, and a surgical instrument controller (surgical instrument instruction input means) 100 which is joystick device transmitting an instruction input signal to the surgical instrument control device 99.

An instruction input signal is transmitted to the surgical instrument control device 99 according to operation of the surgical instrument controller 100 by an operator. The motor box 97 is controlled by the surgical instrument control device 99 based upon the instruction input signal, and behavior of the bending portion 30 of the surgical instrument 12 is performed by the motor box 97. At this time, position/attitude information of the bending portion 30 of the surgical instrument 12 is acquired by the surgical instrument position/attitude information acquisition means 98.

The surgical instrument system 92 performs communication with the endoscopic system 91 regarding the endoscope position/attitude information, and the surgical instrument control device 99 controls the motor box 97 while causing the motor box 97 to cooperate with the endoscope powering means 93. Incidentally, the endoscope control device 95 and the surgical instrument control device 99 are incorporated in one control device 101.

Next, an operation of the endoscopic surgical system with the abovementioned configuration according to the embodiment will be explained. The following behaviors or steps are performed at the time of use of the endoscopic surgical system according to the embodiment.

Step 1: Behavior or actuation of the endoscope control device 95 starts so that an affected area is observed by the endoscope 11.

Step 2: The insertion portion 27 of the surgical instrument 12 is inserted into the channel 20 of the endoscope 11 so that surgical instrument control starts.

Step 3-1: An operator inputs instructions for an insertion operation and a rotation operation of the surgical instrument 12 and for a bending operation of the bending portion 30 of the surgical instrument 12 into the surgical instrument controller 100.

Step 3-2: The surgical instrument controller 100 transmits an instruction input signal to the surgical instrument control device 99. Thereby, the first bending piece 33 and the second bending piece 34 of the bending portion 30 are rotationally driven individually by the bending portion operation mechanism of the motor box 97, so that bending portion 30 of the surgical instrument 12 is operated in a bending manner according to operation of the surgical instrument controller 100. The surgical instrument 12 is driven for rotation and insertion by the surgical instrument operation mechanism of the motor box 97 so that the surgical instrument 12 is operated in a rotating and inserting manner according to operation of the surgical instrument controller 100.

Step 4: At an inserting and rotating operation time of the surgical instrument 12 and at a bending operation time of the bending portion 30 of the surgical instrument 12, position/attitude information of the surgical instrument 12 is acquired by the surgical instrument position/attitude information acquisition means 98 to be transmitted to the surgical instrument control device 99.

Step 5-1: The operator operates the endoscope controller 96 in order to change the visual field of the endoscope 11.

Step 5-2: At this time, the endoscope controller 96 transmits an endoscope instruction input signal to the endoscope control device 95.

Step 6: Thereby, the endoscope position/attitude information acquisition means 94 acquires position/attitude information of the insertion portion 13 of the endoscope 11 to transmit the same to the endoscope control device 95 and the surgical instrument control device 99.

Step 7: The endoscope control device 95 performs calculation based upon the signals received in Steps 5-2 and 6 to control power of the endoscope powering means 93. Thereby, control for adjusting a control signal of the motor box 97 is performed utilizing the position/attitude information from the endoscope position/attitude information acquisition means 94.

Step 8: Thereafter, power generated from the endoscope powering means 93 is transmitted to the insertion portion 13 of the endoscope 11 so that the insertion portion 13 of the endoscope 11 behaves or is actuated in an advancing or retreating direction and/or in a rotating direction.

Step 9: Subsequently, the control device 39 performs calculation based upon the signals received in Steps 3-2, 4, and 6 to control power of the motor box 97.

Step 10: At this time, power generated from the motor box 97 is transmitted to the surgical instrument 12 so that the surgical instrument 12 behaves or is actuated in an inserting and/or rotating manner and the bending portion 30 of the surgical instrument 12 behaves.

The abovementioned Steps 3-1 to 10 configure a series of behaviors. Here, the power control of the motor box 97 in Step 9 is control for cancelling influence to the position/attitude of the surgical instrument 12 within an endoscope image from the endoscope operation in Step 5-1. Thereby, the operator can operate the endoscope 11 and the surgical instrument 12 as devices independent from each other.

Therefore, in the embodiment with the abovementioned configuration, an endoscopic surgical system where, when procedure is performed, the endoscope 11 and position/attitude of the surgical instrument 12 within an endoscope image can be operated independently from each other and the position/attitude of the endoscope 11 can be operated without changing a relative position/attitude between an affected area and the surgical instrument 12 can be provided. Thereby, since movement of the endoscope 11 and movement of the surgical instrument 12 can be synchronized with each other, the surgical instrument 12 can always be disposed within the visual field of the endoscope 11 when procedure is performed. Therefore, such a phenomenon that the position of the surgical instrument 12 within the endoscope image completely changes according to movement of the endoscope 11 when the endoscope 11 and the surgical instrument 12 are used in combination in a conventional manner can be prevented from occurring.

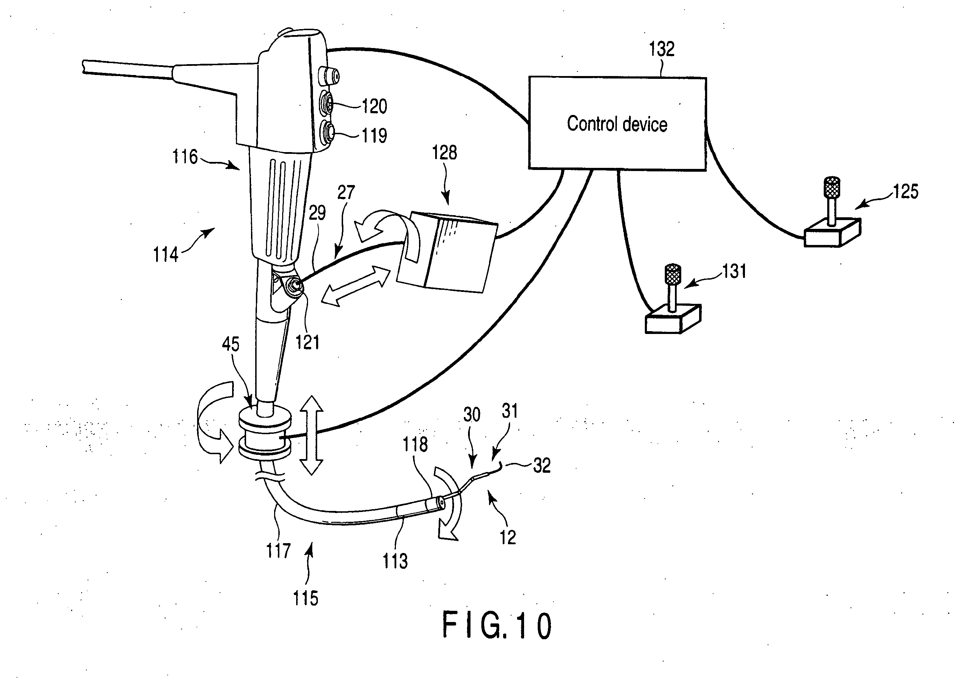

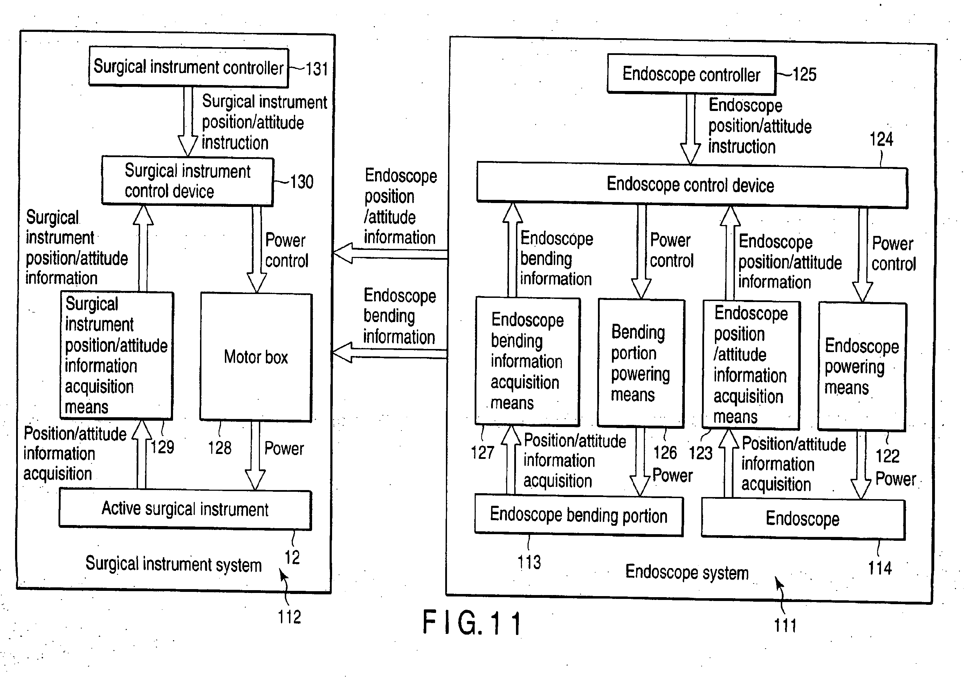

FIGS. 10 and 11 show a third embodiment. FIG. 10 is a schematic configuration diagram of a whole endoscopic surgical system according to the third embodiment and FIG. 11 is a block diagram thereof. Incidentally, in the third embodiment, same parts or portions as those of the first embodiment are attached with same reference numerals, and explanation thereof is omitted.

An endoscopic surgical system according to the embodiment includes an endoscopic system 111 shown in FIG. 11 and a surgical instrument 112 driving the active surgical instrument 12 of the first embodiment. In the endoscopic system 111 of the embodiment, an active endoscope 114 having an active bending portion 113 which can be actively driven by pulling an operation wire (not shown) by driving force from a motor (not shown) is used instead of the endoscope 11 of the manually bending type where the bending portion 16 is operated in a bending manner according to manual operation like the first embodiment (see FIGS. 1 to 4).

As shown in FIG. 10, the active endoscope 114 includes an elongated insertion portion 115 inserted into the body of a patient and an operation section 116 coupled to a proximal end portion of the insertion portion 115. The insertion portion 115 includes an elongated flexible tube portion 117, the active bending portion 113 coupled to a distal end of the flexible tube portion 117, and a distal end hard portion 118 coupled to a distal end of the bending portion 113. The bending portion 113 is configured so as to bent, for example, in four directions of upward and downward directions and leftward and rightward directions.

A distal end face of the distal end hard portion 118 is provided with, for example, one observation window portion 18, two illumination window portions 19a and 19b, an opening of one channel 20 for surgical instrument insertion, and one gas-feeding and water-feeding nozzle 21 (which are shown in FIG. 1B, respectively) like the first embodiment. An imaging section provided with an optical system such as an objective lens (not shown) and an imaging element such as CCD is disposed inside the observation window portion 18. A lesion or the like within the body cavity is imaged by the imaging element. An imaging signal obtained at the imaging section of the endoscope 114 is transmitted to a display processor (not shown) through a connection cable to be converted to a video signal and an image imaged at the endoscope 114 is displayed on a display device (not shown) by the video signal.

A gas-feeding and water-feeding button 119, a suction button 120, various switches for imaging operation, and the like are disposed on the operation section 116. Further, a channel port 121 communicating with the channel 20 for surgical instrument insertion is formed near a coupling portion of the operation section 116 and the insertion portion 115. The surgical instrument 12 is inserted into the channel port 121.

The endoscopic system 111 of the embodiment includes the endoscope 114, endoscope powering means 122, endoscope position/attitude information acquisition means 123 acquiring position/attitude information of the endoscope 114, an endoscope control device (endoscope control means) 124, an endoscope controller (endoscope instruction input means) 125, a bending portion powering means 126, and endoscope bending information acquisition means 127.

The endoscope powering means 122 performs behavior or actuation for straightly moving and rotating the insertion portion 115 of the endoscope 114, namely, performs inserting and detaching operations of the insertion portion 115 for moving the insertion portion 115 of the endoscope 114 in an axial direction of the insertion portion 115 and rotating operation of the insertion portion 115 of the endoscope 114 in a spinning direction of the insertion portion 115. The endoscope position/attitude information acquisition means 123 includes for example, the insertion amount detecting section 50 (see FIG. 3) detecting an insertion amount of the insertion portion 115 of the endoscope 114 and the rotation amount detecting section 51 (see FIG. 3) detecting a rotation amount of the insertion portion 115 of the endoscope 114 in a spinning direction thereof like the first embodiment, and it acquires position/attitude information of the insertion portion 115 of the endoscope 114.

The endoscope controller 125 comprises, for example, a joystick device. Further, the endoscope control device 124 is connected with the endoscope controller 125, the endoscope powering means 122, and the endoscope position/attitude information acquisition means 123. An operator operates the endoscope controller 125 so that an instruction input signal is transmitted to the endoscope control device 124. The endoscope powering means 122 is controlled based upon the instruction input signal by the endoscope control device 124, so that behavior or actuation for straightly moving and rotating the insertion portion 115 of the endoscope 114 is performed. At this time, position/attitude information of the insertion portion 115 of the endoscope 114 is acquired by the endoscope position/attitude information acquisition means 123.

An instruction input signal is transmitted to the endoscope control device 124 by the joystick device of the endoscope controller 125. The bending portion powering means 126 is controlled based upon the instruction input signal by the endoscope control device 124, and behavior of the bending portion 113 is performed by the bending portion powering means 126. At this time, position/attitude information of the bending portion 113 is acquired by the endoscope bending information acquisition means 127.

The surgical instrument system 112 is provided with the active surgical instrument 12, a motor box 128 with a configuration similar to that of the motor box 28 of the first embodiment, surgical instrument position/attitude information acquisition means 129 for acquiring position/attitude information of the surgical instrument 12, a surgical instrument control device (surgical instrument control means) 130 controlling the endoscope powering means 122 and the motor box 128, and a surgical instrument controller (surgical instrument instruction input means) 131 which is a joystick device transmitting an instruction input signal to the surgical instrument control device 130.

An instruction input signal for operating the bending portion 30 in a bending manner is transmitted to the surgical instrument control device 130 according to operation of the joystick device of the surgical instrument controller 131 by an operator. The motor box 128 is controlled based upon the instruction input signal by the surgical instrument control device 130, and behavior of the bending portion 30 of the surgical instrument 12 is performed by the motor box 128. At this time, position/attitude information of the bending portion 30 of the surgical instrument 12 is acquired by the surgical instrument position/attitude information acquisition means 129.

The surgical instrument system 112 performs communication with the endoscopic system 111 regarding the endoscope position/attitude information and the surgical instrument control device 130 controls the motor box 128 while causing the motor box 128 to cooperate with the endoscope powering means 122. Incidentally, the endoscope control device 124 and the surgical instrument control device 130 are incorporated into one control device 132.

Next, an operation of the endoscopic surgical system with the abovementioned configuration according to the embodiment will be explained. The following behaviors or steps are performed at the time of use of the endoscopic surgical system according to the embodiment.

Step 1: Behavior of the endoscope control device 124 starts so that an affected area is observed by the endoscope 114.

Step 2: The insertion portion 27 of the surgical instrument 12 is inserted into the channel 20 of the endoscope 114 so that surgical instrument control is started.

Step 3-1: An operator inputs instructions for performing an inserting operation and a rotating operation of the surgical instrument 12 and for operating the bending portion 30 of the surgical instrument 12 in a bending manner into the surgical instrument controller 131.

Step 3-2: The surgical instrument controller 131 transmits an instruction input signal to the surgical instrument control device 130. Thereby, the first bending piece 33 and the second bending piece 34 of the bending portion 30 are rotationally driven individually by the bending portion operation mechanism of the motor box 128, so that the bending portion 30 of the surgical instrument 12 is operated in a bending manner according to operation of the surgical instrument controller 131. The surgical instrument 12 is driven for rotation and insertion by the surgical instrument operation mechanism of the motor box 128, so that the surgical instrument 12 is operated for rotation and insertion according to operation of the surgical instrument controller 131.

Step 4: At this time, the surgical instrument position/attitude information acquisition means 129 acquires position/attitude information of the surgical instrument 12 to transmit the same to the surgical instrument control device 130.

Step 5-1: The operator operates the endoscope controller 125 in order to change the visual field of the endoscope 114.

Step 5-2: The endoscope controller 125 transmits an endoscope instruction input signal to the endoscope control device 124.

Step 6: The endoscope position/attitude information acquisition means 123 acquires position/attitude information of the insertion portion 115 of the endoscope 114 to transmit the same to the endoscope control device 124 and the surgical instrument control device 130.

Step 7: The endoscope bending information acquisition means 127 acquires bending information of the endoscope bending portion 113 to transmit the same to the endoscope control device 124 and the surgical instrument control device 130.

Step 8: The endoscope control device 124 performs calculation based upon the signals received in Steps 5, 6, and 7 to control power of the endoscope powering means 122 and control power of the bending portion powering means 126. Thereby, control for adjusting a control signal of the motor box 128 is performed utilizing the position/attitude information from the endoscope position/attitude information acquisition means 123.

Step 9: Power generated from the endoscope powering means 122 is transmitted to the insertion portion 115 of the endoscope 114 so that the insertion portion 115 of the endoscope 114 behaves or actuated in an advancing or retreating direction and/or in a rotating direction.

Step 10: Thereafter, power generated from the bending portion powering means 126 is transmitted to the endoscope bending portion 113 so that the bending portion 113 of the endoscope 114 behaves or is actuated in a bending manner.

Step 11: The surgical instrument control device 130 performs calculation based upon the respective signals received at the abovementioned Steps 3-2, 4, and 6 to control power of the motor box 128.

Step 12: Power generated from the motor box 128 is transmitted to the surgical instrument 12 so that the bending portion 30 of the surgical instrument 12 behaves.

The abovementioned Steps 3-1 to 10 configure a series of behaviors. Here, the control in Step 9 is control for cancelling influence to the position/attitude of the surgical instrument 12 within an endoscope image from the endoscope operation in Step 5-1. Thereby, the operator can operate the endoscope 114 and the surgical instrument 12 as devices independent from each other.

In the embodiment with the abovementioned configuration, an endoscopic surgical system where, when procedure is performed, the endoscope 114 and position/attitude of the surgical instrument 12 within an endoscope image can be operated independently from each other, and position/attitude of the endoscope 114 can be operated without changing a relative position/attitude between an affected area and the surgical instrument 12 can be provided. Thereby, when the endoscope 114 moves, the active driving type surgical instrument 12 for a soft endoscope behaves in a linking manner with the movement of the endoscope 114, so that a relative positional deviation occurring between the endoscope 114 and the active driving type surgical instrument 12 for a soft endoscope, which is unintended by an operator, is prevented from occurring. That is, when the endoscope 114 moves, the active driving type surgical instrument 12 for a soft endoscope seems in a fixed state within a soft endoscope image.

Further, the present disclosure is not limited to the abovementioned embodiments and it can be implemented in variously modified states without departing from the gist of the invention.

Next, other characteristic technical items of the present application will be additionally described below.

Note

(Additional Item 1) An active driving type surgical instrument apparatus for a soft endoscope comprising: a surgical means for surgery on an analyte; powering means for actively actuating the surgical means; control means for controlling the powering means; a soft endoscope for inserting the surgical means to use the same; and measuring means for measuring position information of the endoscope, wherein the position information measured by the measuring means is taken in for controlling performed by the control means. (Additional Item 2) The active driving type surgical instrument apparatus for a soft endoscope according to additional item 1, wherein the measuring means is a mouthpiece which detects an insertion amount and a rotation amount of the endoscope.

(Additional Item 3) An endoscope apparatus comprising: a surgical means which can be inserted into a channel of an endoscope and treats an analyte; position information measuring means which measures position information of the endoscope; and control means which controls the position of the surgical means based upon the measured position information.

(Additional Item 4) An endoscope apparatus comprising: a surgical means which can be inserted into a channel of an endoscope and treats an analyte; position information measuring means which measures position information of the surgical means; and control means which controls the position of the endoscope based upon the measured position information.

(Additional Item 5) An endoscopic surgical system comprising: an endoscope; surgical means which can be inserted into a channel of the endoscope or a channel of an overtube attached to the endoscope and treats an analyte; surgical instrument position/attitude information acquisition means for acquiring position/attitude information of the surgical means; surgical instrument powering means which actively actuates the surgical means; control means for controlling the surgical instrument powering means; and surgical instrument instruction input means which transmits an instruction input signal to the control means, wherein endoscope position/attitude information acquisition means for acquiring position/attitude information of the endoscope is provided, and the control means utilizes the position/attitude information.

(Additional Item 6) An endoscopic surgical system comprising: an endoscopic system including an endoscope, endoscope position/attitude information acquisition means for acquiring position/attitude information of the endoscope, endoscope powering means which straightly moves or rotates the endoscope, endoscope control means which controls the endoscope powering means, and endoscope instruction input means for transmitting an instruction input signal to the endoscope control means; and a surgical system including surgical means which can be inserted into a channel of the endoscope or a channel of an overtube attached to the endoscope and treats an analyte, surgical instrument position/attitude information acquisition means for acquiring position/attitude information of the surgical means, surgical instrument powering means which actively actuates the surgical means, surgical instrument control means which controls the surgical instrument powering means, surgical instrument instruction input means for transmitting an instruction input signal to the surgical instrument control means, and surgical instrument control means for controlling the endoscope powering means and the surgical instrument powering means, wherein the surgical system performs communication with the endoscopic system regarding the endoscope position/attitude information and the surgical instrument control means controls the surgical instrument powering means while causing the surgical instrument powering means to cooperate with the endoscope powering means.

(Additional Item 7) An endoscopic surgical system comprising: an endoscopic system including an endoscope having an active bending portion, endoscope position/attitude information acquisition means for acquiring position/attitude information of the endoscope, endoscope powering means which straightly moves and rotates the endoscope, endoscope bending information acquisition means for acquiring bending information of the bending portion, bending portion powering means for bending the active bending portion of the endoscope, endoscope controt means which controls the endoscope powering means and the bending portion powering means, and endoscope instruction input means for transmitting an instruction input signal to the endoscope control means; and a surgical system including surgical means which can be inserted into a channel of the endoscope or a channel of an overtube attached to the endoscope and treats an analyte, surgical instrument position/attitude information acquisition means for acquiring position/attitude information of the surgical means, surgical instrument powering means which actively actuates the surgical means, surgical instrument control means which controls the surgical instrument powering means, surgical instrument instruction input means for transmitting an instruction input signal to the surgical instrument control means, and surgical instrument control means for controlling the endoscope powering means and the surgical instrument powering means, wherein the surgical system performs communication with the endoscopic system regarding the endoscope position/attitude information and the surgical instrument control means controls the surgical instrument powering means while causing the surgical instrument powering means to cooperate with the endoscope powering means.

## Claims

1. An endoscopic surgical system comprising:

an endoscope (11) including an insertion portion (13), wherein position/attitude operation of the insertion portion (13) including an insertion operation and a rotation operation of the insertion portion (13) is performable; a surgical instrument (12) configured to be inserted into a channel (20) of the endoscope (11) and configured to treat an analyte; surgical instrument powering means (28) configured to actively actuate the surgical instrument (12) to perform position/attitude operation of the surgical instrument (12) including an insertion operation and a rotation operation of the surgical instrument (12); control means (39) which controls the surgical instrument powering means (28); and surgical instrument instruction input means (40) which transmits an instruction input signal to the control means (39), surgical instrument position/attitude information acquisition means (38) for acquiring position/attitude information of the surgical instrument (12) and for transmitting the position/attitude information of the surgical instrument (12) to the control means (39); and

endoscope position/attitude information acquisition means (44) configured to acquire position/attitude information of the endoscope (12) and configured to transmit the position/attitude information of the insertion portion (13) to the control means (39), wherein the position/attitude information of the insertion portion (13) includes an insertion amount and a rotation amount of the insertion portion (13),

**characterized in that**

the control means (39) includes control signal adjusting means configured to adjust a control signal of the surgical instrument powering means (28) by utilizing the position/attitude information of the endoscope (12) acquired by the endoscope position/attitude information acquisition means (44) when the surgical instrument (12) is inserted into the channel (20) such that the surgical instrument (12) can be operated independently from the endoscope (11) and the position/attitude of the endoscope (11) can be operated without changing a relative position/attitude of the surgical instrument (12) with respect to an affected area.

2. The endoscopic surgical system according to claim 1, **characterized in that**:

the surgical instrument instruction input means (40) is configured for transmitting an instruction rotation amount of the surgical instrument (12) and an instruction insertion amount of the surgical instrument (12) to the control means (39); the endoscope position/attitude information acquisition means (44) comprises an insertion amount detecting section (50) and a rotation amount detecting section (51) for acquiring the position/attitude information of the insertion portion (13), and

the surgical instrument powering means (28) is configured to actuate the surgical instrument (12) in an inserting direction and to rotationally operate the surgical instrument (12);

the control means (39) is configured to control the surgical instrument powering means (28) so as to satisfy the following equations:

$$z = y - x;$$

and

$$\gamma = \beta - \alpha,$$

wherein:

x is an insertion amount of the insertion portion (13) detected by the endoscope position/attitude information acquisition means (44) ;

$\alpha$ is a rotation amount of the insertion portion (13) detected by the endoscope position/attitude information acquisition means (44);

y is an instruction insertion amount of the surgical instrument (12) received from the surgical instrument instruction input means (40);

$\beta$ is an instruction rotation amount of the surgical instrument (12) received from the surgical instrument instruction input means (40);

z is an insertion amount that the surgical instrument (12) is moved by the surgical instrument powering means (28); and

$\gamma$ is a rotation amount that the surgical instrument (12) is rotated by the surgical instrument powering means (28).

**Patentansprüche**

1. Endoskopisches chirurgisches System, das umfasst:

ein Endoskop (11) mit einem Einführabschnitt (13), wobei eine Positions-/Stellungsbetätigung des Einführabschnitts (13) einschließlich einer Einführbetätigung und einer Rotationsbetätigung des Einführabschnitts (13) durchführbar ist;

ein chirurgisches Instrument (12), das dazu eingerichtet ist, in einen Kanal (20) des Endoskops (11) eingeführt zu werden und das dazu eingerichtet ist, einen Analyt zu behandeln;

ein Mittel (28) zum Antreiben des chirurgischen Instruments, das dazu eingerichtet ist, das chirurgische Instrument (12) aktiv zu betätigen, um eine Positions-/Stellungsbetätigung des chirurgischen Instruments (12) einschließlich einer Einführbetätigung und einer Rotationsbetätigung des chirurgischen Instruments (12) durchzuführen;

ein Steuerungsmittel (39), das das Mittel (28) zum Antreiben des chirurgischen Instruments steuert; und

ein Mittel (40) zum Eingeben eines Befehls für das chirurgische Instrument, das ein Befehlseingabesignal an das Steuerungsmittel (39) überträgt,

ein Mittel (38) zum Erfassen einer Positions-/Stellungsinformation für das chirurgische Instrument zum Erfassen einer Positions-/Stellungsinformation des chirurgischen Instruments (12) und zum Übertragen der Positions-/Stellungsinformation des chirurgischen Instruments (12) an das Steuerungsmittel (39); und

ein Mittel (44) zum Erfassen einer Endoskop-Positions-/Stellungsinformation, das dazu eingerichtet ist, eine Positions-/Stellungsinformation des Endoskops (11) zu erfassen und das dazu eingerichtet ist, die Positions-/Stellungsinformation des Einführabschnitts (13) an das Steuerungsmittel (39) zu übertragen, wobei die Positions-/Stellungsinformation des Einführabschnitt (13) einen Einführbetrag und einen Rotationsbetrag des Einführabschnitts (13) enthält,

**dadurch gekennzeichnet, dass**

das Steuerungsmittel (39) ein Steuersignaleinstellmittel umfasst, das dazu eingerichtet ist, ein Steuersignal des Mittels (28) zum Antreiben des chirurgischen Instruments unter Verwendung der durch das Mittel (44) zum Erfassen einer Endoskop-Positions-/Stellungsinformation erfassten Positions-/Stellungsinformation des Endoskops (11) anzupassen, wenn das chirurgische Instrument (12) in den Kanal (20) eingeführt wird, so dass das chirurgische Instrument (12) unabhängig von dem Endoskop (11) betätigt werden kann und die Position/Stellung des Endoskops (11) betätigt werden kann, ohne eine relative Position/Stellung des chirurgischen Instruments (12) bezüglich eines betroffenen Bereichs zu verändern.

2. Endoskopisches chirurgisches System gemäß Anspruch 1, **dadurch gekennzeichnet, dass**:

das Mittel (40) zum Eingeben eines Befehls für das chirurgische Instrument dazu eingerichtet ist, einen Befehlsrotationsbetrag des chirurgischen Instruments (12) und einen Befehlseinführbetrag des chirurgischen Instruments (12) an das Steuerungsmittel (39) zu übertragen;

das Mittel (44) zum Erfassen einer Endoskop-Positions-/Stellungsinformation einen Einführbetragserfassungsabschnitt (50) und einen Rotationsbetragserfassungsabschnitt (51) zum Erfassen der Positions-/Stellungsinformation des Einführabschnitts (13) umfasst und das Mittel (28) zum Antreiben des chirurgischen Instruments dazu eingerichtet ist, das chirurgische Instrument (12) in einer Einführrichtung zu betätigen und das chirurgische Instrument (12) drehend zu betätigen;

das Steuerungsmittel (39) dazu eingerichtet ist, das Mittel (28) zum Antreiben des chirurgischen Instruments so zu steuern, dass die folgenden Gleichungen erfüllt sind:

$$z = y - x;$$

und

$$\gamma = \beta - \alpha,$$

wobei:

x ein durch das Mittel (44) zum Erfassen einer Endoskop-Positions-/Stellungsinformation erfasster Einführbetrag des Einführabschnitts (13) ist;

$\alpha$ ein durch das Mittel (44) zum Erfassen einer Endoskop-Positions-/Stellungsinformation erfasster Rotationsbetrag des Einführabschnitts (13) ist;

y ein von dem Mittel (40) zum Eingeben eines Befehls für das chirurgische Instrument empfangener Befehlseinführbetrag des chirurgischen Instruments (12) ist;

$\beta$ ein von dem Mittel (40) zum Eingeben eines Befehls für das chirurgische Instrument empfangener Befehlsrotationsbetrag des chirurgischen Instruments (12) ist;

z ein Einführbetrag ist, um den das chirurgische Instrument (12) durch das Mittel (28) zum Antreiben des chirurgischen Instruments bewegt wird; und

$\gamma$ ein Rotationsbetrag ist, um den das chirurgische Instrument (12) durch das Mittel (28) zum Antreiben des chirurgischen Instruments gedreht wird.

## Revendications

1. Système chirurgical endoscopique comprenant :

un endoscope (11) incluant une partie d'insertion (13), dans lequel une opération de position/attitude de la partie d'insertion (13) incluant une opération d'insertion et une opération de rotation de la partie d'insertion (13) est réalisable ;

un instrument chirurgical (12) configuré pour être inséré dans un canal (20) de l'endoscope (11) et configuré pour traiter un analyte ;

un moyen (28) d'alimentation d'instrument chirurgical configuré pour actionner de manière active l'instrument chirurgical (12) pour réaliser une opération de position/attitude de l'instrument chirurgical (12) incluant une opération d'insertion et une opération de rotation de l'instrument chirurgical (12) ;

un moyen de commande (39) qui commande le moyen d'alimentation (28) d'instrument chirurgical ; et

un moyen (40) d'entrée d'instruction d'instrument chirurgical qui transmet un signal d'entrée d'instruction au moyen de commande (39),

un moyen (38) d'acquisition d'informations de position/attitude d'instrument chirurgical destiné à acquérir des informations de position/attitude de l'instrument chirurgical (12) et destiné à transmettre les informations de position/attitude de l'instrument chirurgical (12) au moyen de commande (39) ; et

un moyen (44) d'acquisition d'informations de position/attitude d'endoscope configuré pour acquérir des informations de position/attitude de l'endoscope (12) et configuré pour transmettre les informations de position/attitude de la partie d'insertion (13) au moyen de commande (39), dans lequel les informations de position/attitude de la partie d'insertion (13) contiennent une quantité d'insertion et une quantité de rotation de la partie d'insertion (13),

**caractérisé en ce que**

le moyen de commande (39) comprend un moyen d'ajustement de signal de commande configuré pour ajuster un signal de commande du moyen d'alimentation (28) d'instrument chirurgical en utilisant les informations de position/attitude de l'endoscope (12) acquises par le moyen (44) d'acquisition d'informations de position/attitude d'endoscope lorsque l'instrument chirurgical (12) est inséré dans le canal (20) d'une manière telle que l'instrument chirurgical (12) peut être actionné indépendamment de l'endoscope (11) et la position/attitude de l'endoscope (11) peut être actionnée sans changer une position/attitude relative de l'instrument chirurgical (12) par rapport à une zone affectée.

2. Système chirurgical endoscopique selon la revendication 1, **caractérisé en ce que** :

le moyen (40) d'entrée d'instruction d'instrument chirurgical est configuré pour transmettre une instruction de quantité de rotation de l'instrument chirurgical (12) et une instruction de quantité d'insertion de l'instrument chirurgical (12) au moyen de commande (39) ;

le moyen (44) d'acquisition d'informations de position/attitude d'endoscope comprend une section (50) de détection de quantité d'insertion et une section (51) de détection de quantité de rotation destinées à acquérir les informations de position/attitude de la partie d'insertion (13), et

le moyen d'alimentation (28) d'instrument chirurgical est configuré pour actionner l'instrument chirurgical (12) dans une direction d'insertion et pour actionner l'instrument chirurgical (12) en rotation ;

le moyen de commande (39) est configuré pour commander le moyen d'alimentation (28) d'instrument chirurgical de façon à satisfaire les équations suivantes :

$$z = y - x \; ;$$

et

$$\gamma = \beta - \alpha,$$

dans lesquelles :

x représente une quantité d'insertion de la partie d'insertion (13) détectée par le moyen (44) d'acquisition d'informations de position/attitude d'endoscope ;

α représente une quantité de rotation de la partie d'insertion (13) détectée par le moyen (44) d'acquisition d'informations de position/attitude d'endoscope ;

y représente une instruction de quantité d'insertion de l'instrument chirurgical (12) reçue du moyen (40) d'entrée d'instruction d'instrument chirurgical ;

β représente une instruction de quantité de rotation de l'instrument chirurgical (12) reçue du moyen (40) d'entrée d'instruction d'instrument chirurgical ;

z représente une quantité d'insertion de laquelle l'instrument chirurgical (12) est déplacé par le moyen d'alimentation (28) d'instrument chirurgical ; et

y représente une quantité de rotation de laquelle l'instrument chirurgical (12) est tourné par le moyen d'alimentation (28) d'instrument chirurgical.

FIG.1A

FIG.1B

FIG.2

FIG.3

Control device

40

| Joystick device |

Surgical instrument
position/attitude instruction          39

| Control device |

Surgical instrument
position/attitude
information

Power control

Endoscope
position/attitude
information

38 —

| Surgical instrument
position/attitude
information
acquisition means |

| Motor box | — 28

| Endoscope
position/attitude
information acquisition
means | — 44

Position/attitude
information
acquisition

Power

Position/attitude
information
acquisition

| Surgical instrument |

| Endoscope |

12

11

# FIG. 4

45

72

76

71

74 73

75

11 15

13

39

Control device

# F I G. 5

45

81 82

11 15

13

39

Control device

# F I G. 6

FIG.7

EP 2 147 630 B1

F I G. 8

FIG.9

FIG.10

EP 2 147 630 B1

FIG.11

FIG.12A

FIG.12B

FIG.13A

FIG.13B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005137701 A
- US 5876325 A
- EP 2064984 A